**Europäisches Patentamt**

**(19)** **European Patent Office**

**Office européen des brevets**

**(11)** Publication number : **0 643 971 A2**

## **(12)** EUROPEAN PATENT APPLICATION

**(21)** Application number : **94306122.6**

**(22)** Date of filing : **19.08.94**

**(51)** Int. Cl.<sup>6</sup>: **A61K 38/02, // (A61K38/02, 31:135, 31:475, 31:54)**

**(30)** Priority : **25.08.93 US 111680**

**(43)** Date of publication of application :
**22.03.95 Bulletin 95/12**

**(84)** Designated Contracting States :
**AT BE CH DE DK ES FR GB GR IE IT LI LU NL PT SE**

**(71)** Applicant : **ELI LILLY AND COMPANY
Lilly Corporate Center
Indianapolis Indiana 46285 (US)**

**(72)** Inventor : **Peery, Robert Brown
372 Sycamore Street
Brownsburg, Indiana 46112 (US)**
Inventor : **Skatrud, Paul Luther
2412 Lake Crossing
Greenwood, Indiana 46143 (US)**

**(74)** Representative : **Tapping, Kenneth George et al
Lilly Industries Limited
European Patent Operations
Erl Wood Manor
Windlesham Surrey GU20 6PH (GB)**

**(54)** Antifungal compositions consisting of an antifungal agent, e.g. antibiotic R-106l, and a fungal MDR inhibitor.

**(57)** The invention provides, among others, a method of increasing the sensitivity of a fungal cell to an antifungal agent by administering an antifungal agent in combination with a potentiating agent. The invention also provides a method of testing for the ability of an agent to potentiate the activity of an antifungal compound.

EP 0 643 971 A2

The incidence of serious, life-threatening, fungal infections is increasing at an alarming rate. The number of *Candida albicans* bloodstream infections in non-teaching hospitals increased by 370% between 1980 and 1990. At the same time the incidence of bloodstream infections by C. *albicans* in teaching hospitals increased by 487% in the same time period. With the exception of coagulase negative staphylococci, statistically, *C. albicans* represents the fastest growing area of concern in hospital acquired bloodstream infections (Banerjee *et al.*, 1991, American Journal of Medicine 91(3B):86S-89S).

The rising incidence of nosocomial fungal infections is being fueled by changing medical practices, including invasive surgical procedures addressing such problems as joint replacement and open heart surgery, increased use of cancer chemotherapy, and the AIDS epidemic. All of these processes compromise the immune system and provide an environment in which fungal infections can be established.

Approximately 90% of nosocomial fungal infections are represented by species of *Candida*. The remaining fungal infections are for the most part comprised of *Aspergillus* species, *Cryptococcus* species, and *Pneumocystis carrinii*. Unfortunately, diagnosis of fungal infections is difficult and time consuming. Rather than waiting for the results of diagnostic tests, empirical antifungal therapy must frequently be initiated with a compound such as amphotericin B (which has a high toxicity liability) or one of the azoles (to which fungal species are rapidly developing resistance). Thus, having an antifungal with a broad spectrum of activity coupled with a low toxicity profile would be very desirable.

The discovery and development of antifungal compounds for specific fungal species has met with some degree of success. Because *Candida* species represent the majority of fungal infections, screens for new antifungal compounds have been designed to discover anti-*Candida* compounds. During product development, anti-fungal activity has generally been optimized based on activity against *Candida albicans*. As a consequence, these anti-*Candida* compounds frequently do not possess clinically significant activity against other fungal species. However, it is interesting to note that at higher concentrations some of these compounds are able to kill other fungal species, suggesting that the antifungal target is present in these organisms as well. In retrospect, such results indicate that these organisms possess some natural mechanism of resistance that permit them to survive in clinically relevant concentrations of several antifungal compounds. Such a general mechanism of resistance to antifungal compounds has remained undescribed in the fungi for decades.

The problem of multiple drug resistance (mdr) mediated by the *mdr* gene was initially recognized during the course of developing regimens for cancer chemotherapy. A multiple drug resistant cancer cell line exhibits resistance to high-levels of a large variety of cytotoxic compounds. Frequently these cytotoxic compounds will have no common structural features nor will they interact with a common target within the cell. Resistance to these cytotoxic agents is mediated by an outward directed, ATP dependent pump. By this mechanism toxic levels of a particular compound are not allowed to accumulate within the cell.

In addition to efflux, there is also evidence that influx may also be reduced in cells expressing *mdr* (Gottesman and Pastan, 1993, Annual Review of Biochemistry 62:385-427). A rather large family of *mdr* genes have been characterized which include a number of quite divergent organisms. To date the list of organisms that posses *mdr*-like function include numerous bacterial species, the fruit fly *Drosophila melanogaster*, *Plasmodium falciparum*, *Homo sapiens*, the yeast *Saccharomyces cerevisiae*, *Caenorhabditis elegans*, *Leishmania donovanii*, marine sponges, and the plant *Arabidopsis thaliana*.

Decades of research have been expended on describing the multiple drug resistance phenotype in human cancer cells. Extensive searches have revealed several classes of compounds that are able to reverse the mdr phenotype of multiple drug resistant cancer cell lines rendering them susceptible to the effects of cytotoxic compounds (for example, calcium channel blockers, anti-arrhythmics, antihypertensives, antibiotics, antihistamines, immunosuppressants, steroid hormones, modified steroids, lipophilic cations, diterpenes, detergents, antidepressants, antipsychotics, as well as many other hydrophobic amphipathic compounds and their analogs, (Gottesman and Pastan, 1993, Annual Review of Biochemistry 62:385-427)). Such compounds are termed *mdr* inhibitors. The clinical application of human *mdr* inhibitors to cancer chemotherapy has become an area of intensive focus for research.

Recently, the existence of multiple drug resistance (*mdr*)-related genes in a non-pathogenic as well as in key opportunistic pathogenic fungi has been discovered. These genes have been termed "*mdr*-like" because of the homology that exists between the proteins encoded by these genes and the human *mdr*-1 gene product.

With the discovery of *mdr*-like genes in a variety of fungi, including key pathogenic fungi, such as *Aspergillus flavus*, *Aspergillus fumigatus*, and *Cryptococcus neoformans*, the prospect of using combination therapy involving an antifungal agent possessing a proven spectrum of activity, with a fungal mdr inhibitor becomes a reality.

The combination therapy approach allows an extension of the spectrum of antifungal activity for a given antifungal compound which previously had only demonstrated limited clinically relevant antifungal activity. Similarly, compounds with demonstrated antifungal activity can also be potentiated by a fungal mdr inhibitor such that the antifungal activity of these compounds is extended to previously resistant species.

One aspect of the invention provides a method of increasing the sensitivity of a fungal cell to an antifungal agent comprising administering to said cell an effective amount of each of (i) an antifungal agent and (ii) a potentiating agent.

In another aspect, the present invention provides a pharmaceutical formulation comprising an effective amount of each of (i) an antifungal agent and (ii) a potentiating agent; and pharmaceutically acceptable excipients.

In a further aspect, the invention provides an assay method for determining the antifungal-potentiating effect of a compound on an antifungal agent exposed to a fungal cell comprising:

a) growing a culture of said fungal cell in the presence of (i) an antifungal compound to which said fungal cell is resistant and (ii) a compound suspected of possessing antifungal-potentiating properties; and

b) measuring the antifungal-potentiating effect of said compound on the antifungal agent.

The term "subject" refers herein to an organism, organ, organ system or cell line of an organism, which has fungal cell line(s) living therein. As such, the subject may be treated in vitro or in vivo. The subject is desirably a mammal, to include a human patient with in vivo treatment being undertaken.

The term "antifungal agent" or "antifungal compound" refers herein to a compound or composition which is cytostatic or cytotoxic towards fungal cells.

The terms "potentiating agent", "potentiating", "potentiate", or "potentiated" and so forth refer herein to the property of a compound, compounds, or composition to enhance the activity of an antifungal agent or antifungal compound to a fungal cell.

The terms "enhanced activity" and "enhance the activity" refer to the ability of an antifungal agent or antifungal compound to exhibit cytostatic or cytotoxic effects either at a concentration lower than at which it was static or toxic in the absence of the potentiating agent; or is static or toxic to a fungal cell otherwise resistant to said antifungal agent or antifungal compound in the absence of said potentiating agent.

The terms "resistant" or "resistance" refer to the ability of an organism to grow in the presence of an agent which is cytotoxic to related organisms.

The terms "antifungal activity" or "antifungal effect" refer to the cytostatic or cytotoxic effect of an antifungal agent or antifungal compound on a fungal cell.

The term "administering" and so forth refers herein to suitably providing an amount of antifungal agent or antifungal compound or composition thereof in vitro or in vivo to a cell so that a prophylactic or therapeutic effect might result. The administering can be by mere contact of the components with a cell line, or can be by conventional dosage formats as for a mammalian subject. The conventional dosage formats include the ingestion of an oral or sublingual dosage form such as a powder, sample of beads, tablet, capsule, syrup, the injection of an intravenous solution or colloidal mixture, the application of a transdermal or other external preparation such as a solution, creme, gel or other ointment, and/or the implantation of a rapid or sustained release device.

The antifungal agent and the potentiating agent may be administered either (a) simultaneously in time (optionally by formulating the two together in a common carrier), or (b) at different times during the course of a common treatment schedule. In the latter case, the two compounds are administered at times sufficiently close for the potentiating agent to enhance the antifungal activity of the antifungal agent on the fungal cells. In addition, one or more antifungal agents and one or more potentiating agents may be present in a single formulation.

The potentiating agent is administered in an amount effective to reduce the amount of the antifungal agent required to render the fungal cell susceptible to the amount of antifungal agent administered. The amount of antifungal compound to be employed in the method of the present invention depends on the particular fungal organism to be controlled, the particular environment in which it is to be administered, the antifungal agent chosen, and the particular potentiating agent used.

The composition of antifungal agent(s) and potentiating agent(s), either alone or in combination, can be formulated according to known methods to prepare pharmaceutically useful compositions, whereby the antifungal agent(s) and potentiating agent(s), either alone or in combination, is combined in admixture with a pharmaceutically acceptable carrier vehicle. Suitable carrier vehicles and their formulation, inclusive of other human proteins, e.g., human serum albumin, are described for example, in Remington's Pharmaceutical Sciences 16th ed., 1980, Mack Publishing Co., edited by Oslo et al.. Such compositions will contain an effective amount of antifungal agent and potentiating agent, either alone or in combination, together with a suitable amount of carrier vehicle to prepare pharmaceutical compositions suitable for effective administration to the host. Such compositions can be administered orally, topically, parentally, or by nasal or opthamalic routes, or by other means that ensure delivery of the antifungal agent and potentiating agent to the site of infection in an effective form.

The following examples are intended to assist in further understanding of the invention. Particular materials employed, species and conditions are intended to be further illustrative of the invention and not limiting the reasonable scope thereof.

## Example 1

A clinical isolate of *Aspergillus fumigatus*, strain 10AF (Eli Lilly and Company) was used in antifungal susceptibility/potentiation tests. Spores of *A. fumigatus* strain 10AF were prepared by inoculating Potato dextrose agar (Difco Laboratories, Detroit, MI) with spores from long term storage. These plates were incubated at 35°C for 24 hours and then an additional one to two days at room temperature until sporulation was evident. Spores were harvested by flooding the plates with a 0.05% Tween 80 solution and gently scraping the surface of the plate to suspend the spores. Spores were harvested by centrifugation and resuspended in a cold storage preservation medium containing 50 g/L lactose, 100 ml/L glycerol, and 850 ml water. Spore suspensions prepared in this manner remained viable for several months when stored at -70°C.

Approximately $1 \times 10^6$/ml *Aspergillus fumigatus* strain 10AF spores were suspended in autoclaved Trypticase Soy agar (cooled to approximately 50°C) and 15 ml of the suspension were delivered to each of several petri plates. The agar surfaces of these petri plates were allowed to dry in a biohazard hood.

The antifungal compound R106I was dissolved in 100% ethanol at a concentration of either 1 or 7 mg/ml. The antifungal compound R106I is disclosed in United States Patent No. 5,057,493, which is hereby incorporated herein by reference. Twenty μl of the 1 mg/ml R106I solution was delivered to an antibiotic susceptibility test disc (Difco Laboratories, Detroit, MI). After addition of the antibiotic solution the discs were allowed to air dry in a biohazard hood. When dry, the discs were placed on the surface of the petri plates containing the *Aspergillus fumigatus* spores.

Compounds to be tested for the potentiation of the antifungal activity of R106I (i.e., putative potentiating agent) against *Aspergillus* fumigatus were dissolved in dimethylsulfoxide (DMSO). The amount of compound added to the DMSO depended on the solubility of the individual compound to be tested. Twenty μl of the suspensions containing the compounds to be tested were delivered to an antibiotic susceptibility test disc (Difco Laboratories, Detroit, MI). The discs containing the test compounds were allowed to air dry in a biohazard hood. These discs were then placed on the surface of the dried petri plates containing the *Aspergillus fumigatus* spores approximately 2 cm from the antifungal containing disc. Petri plates containing the two discs were incubated at 35°C overnight. The next morning the petri plates were examined for zones of growth inhibition around the discs.

Table 1 provides a list of compounds (i.e., putative potentiating agents) and the results obtained when these compounds were tested by the above method for the ability to potentiate the activity of R106I against *Aspergillus fumigatus*. At the above indicated concentrations of R106I no significant zone of growth inhibition was observed around discs containing R106I. In Table 1, a result of "+" indicates that a zone of growth inhibition was present on the test plate and, therefore, that the compound contained in the disc adjacent to the antibiotic disc, potentiated the antifungal activity of the antifungal compound R106I. A result of "-" indicates that a zone of growth inhibition was not present on the test plate and, therefore, the compound tested did not potentiate the antifungal activity of the antifungal compound R106I. Table 1 also provides a short description for a number of the compounds tested for the ability to potentiate the antifungal activity of R106I.

## Table 1

| Compound Tested | Result | Comments |
|---|---|---|
| Verapamil[1]<br>300 mg/disc[2] | + | Ca$^{++}$ channel blocker; human mdr inhibitor |
| Vindolin<br>420 mg/disc[2]<br><br>860 mg/disc[3] | –<br><br>+ | Antineoplastic agent; human mdr inhibitor |
| Trifluoroperazine[1]<br>440 mg/disc[1] | + | Antipsychotic; Tranquilizer; human mdr blocker |

[1]Available from the Sigma Chemical Company, St. Louis MO[2]

R106I concentration = 20 mg/disc

[3] R106I concentration = 140 mg/disc

### Example 2

Protoplasts of *Penicillium chrysogenum* (Eli Lilly and Co.) were formed with Novozyme 234 in substantial accordance with the procedure described for *Cephalosporium acremonium* by Queener *et al.*, 1985 Microbiology 1985, American Society for Microbiology, pp 468-472, and by Skatrud *et al.*, 1987, Current Genetics, 12:337-348. During formation of the *P. chrysogenum*, protoplasts and subsequent cell wall regeneration, sucrose was used as an osmotic stabilizer rather than sodium chloride as described in the above references. Petri plates were prepared that contained an agar layer consisting of: yeast nitrogen base without amino acids and ammonium sulfate (1.7 g/L) (Difco Laboratories, Detroit, MI); ammonium sulfate (5 g/L); and sucrose (125 g/L); trifluoroperazine (final concentration 250 mM; Sigma Chemical Co. Catalog #T-8516); and cadmium chloride (final concentration 3 mM/ml). Approximately 3 X 10$^7$ *Penicillium chrysogenum* protoplasts were delivered to the surface of the solidified agar in a 4 ml soft agar overlay consisting of the following ingredients: yeast nitrogen base without amino acids and ammonium sulfate (1.7 g/L) (Difco Laboratories, Detroit, MI); ammonium sulfate (5 g/L); sucrose (125 g/L); and agar (4 g/L). The plates were incubated overnight at 15°C.

The next morning hygromycin B (final concentration 250 mg/ml; Boehringer Mannheim Catalog #843 555) was added to the plates in a 4 ml soft agar overlay consisting of yeast nitrogen base without amino acids and ammonium sulfate (1.7 g/L) (Difco Laboratories, Detroit, MI); ammonium sulfate (5 g/L); sucrose (125 g/L); and agar (4 g/L). The plates were incubated at 28°C for 48 hours. Further tests were conducted wherein the concentration of trifluoroperazine and hygromycin B was varied as shown in Table 2.

The results of this test are presented in Table 2. These results indicate that the toxic effect of hygromycin B on *Penicillium chrysogenum* was potentiated by the presence of triflouroperazine. In Table 2, a result of "+" indicates growth of the *Penicillium chrysogenum* cells, and therefore, that the toxic effect of hygromycin B was not potentiated by TFP. A result of "-" indicates no growth of the *Penicillium chrysogenum* culture, and therefore, that the toxic effect of hygromycin B was potentiated by trifluoroperazine.

**Table 2**

| Penicillium Chrysogenum Growth | | |
|---|---|---|
| Hygromycin B (mg/ml) | TFP (μM) | Growth |
| 0 | 500 | + |
| 0.250 | 250 | - |
| 1 | 500 | - |
| 2 | 500 | - |
| 3 | 500 | - |
| 4 | 500 | - |
| 5 | 500 | - |
| 5 | 0 | + |

## Claims

1. A method of increasing the sensitivity of a fungal cell to an antifungal agent comprising administering to said cell an effective amount of each of (i) an antifungal agent and (ii) a potentiating agent.

2. A method of Claim 1 wherein the antifungal agent is R-106I.

3. A method of Claim 1 or 2 wherein the potentiating agent is a fungal mdr inhibitor.

4. A method of Claims 1, 2, or 3 wherein the potentiating agent is selected from the group consisting of verapamil, vindolin, and trifluoroperazine.

5. A pharmaceutical formulation comprising an effective amount of each of (i) an antifungal agent and (ii) a potentiating agent; and pharmaceutically acceptable excipients.

6. A pharmaceutical formulation of Claim 5 wherein the antifungal agent is R-106I.

7. A pharmaceutical formulation of Claim 5 or 6 wherein the potentiating agent is a fungal mdr inhibitor.

8. A pharmaceutical formulation of Claim 5, 6, or 7 wherein the potentiating agent is selected from the group consisting of verapamil, vindolin, and trifluoroperazine.

9. A pharmaceutical formulation as claimed in any one of Claims 5-8 for use in treating fungal infections.

10. A assay method for determining the antifungal-potentiating effect of a compound on an antifungal agent exposed to a fungal cell comprising:
   a) growing a culture of said fungal cell in the presence of (i) an antifungal compound to which said fungal cell is resistant and (ii) a compound suspected of possessing antifungal-potentiating properties; and
   b) measuring the antifungal-potentiating effect of said compound on the antifungal agent.